# EUROPEAN PATENT SPECIFICATION

(11) **EP 0 969 778 B1**
(45) Date of publication and mention of the grant of the patent: **22.09.2004**
(21) Application number: 98912639.6
(22) Date of filing: 09.04.1998
(51) Int. Cl.: A61F 2/06

(54) **ENDOVASCULAR GRAFT FOR REPAIRING ABDOMINAL AORTIC ANEURYSMS**
ENDOVASKULÄRES IMPLANTAT ZUM AUSBESSERN EINES ANEURYSMAS DER ABDOMINALAORTA
GREFFE ENDOVASCULAIRE POUR REPARER DES ANEVRISMES DE L'AORTE ABDOMINALE

(30) Priority: 10.04.1997 GR 97100134
(43) Date of publication of application: 12.01.2000
(73) Proprietor: WILLIAM COOK, EUROPE A/S, 4632 Bjaeverskov (DK); Konstantinos, Papazoglou O., 552 36 Thessaloniki (GR)
(72) Inventor: PAPAZOGLOU, Konstatinos, GR-552 36 Thessaloniki (GR)
(74) Representative: Johnston, Kenneth Graham
(86) International application number: PCT/IB1998/000530
(87) International publication number: WO 1998/044870

(56) References cited:
- EP-A- 0 686 379
- EP-A- 0 698 380
- EP-A- 0 765 643
- WO-A-95/21592
- WILSON G J ET AL: "A SELF-EXPANDING BIFURCATED ENDOVASCULAR GRAFT FOR ABDOMINAL AORTIC ANEURYSM REPAIR" ASAIO JOURNAL, vol. 42, no. 5, September 1996, pages M386-M393, XP000683606

## Description

The present invention relates to grafts for repairing aortic aneurysms.

The endovascular approach to aneurysms of the abdominal aorta is a new technique for the therapy of aneurysms by the placement of grafts which are transferred to the position of placement by means of the vascular lumen from easily anatomically approachable regions, thus avoiding the need for massive surgical operations. For the effective therapy of an aneurysm by this technique, it is necessary to have good circular application (contact) of the endovascular graft with the healthy, nondistended part of the blood vessel, so as to have complete exclusion of the distended arterial lumen by the pressure of the systemic arterial circulation, since the blood will now come out through the graft which substitutes for the vascular lumen. Furthermore, the endovascular graft must have a small starting-off diameter, which will allow its easy introduction and advancement from the entrance blood vessel to the position of placement, as well as its easy technical positioning. In abdominal aortic aneurysms there usually is a central neck region of the healthy blood vessel having a normal diameter underneath the kidney arteries (at the point where the graft is surgically attached even by the classical operation). However often the aortic distention is peripherally extended up the point of the aortic bifurcation at the two iliac arteries. This fact excludes the possibility of placing an endovascular tube graft due to the absence of a healthy peripheral contact point (neck). This often creates the necessity for the placement of endovascular grafts which can be attached to healthy regions of the iliac arteries more peripherally positioned to the distended aortic bifurcation with the simultaneous branching of the aortic blood flow to two cylinders of effluence. The systems that till now have been presented for the placement of aortic stent grafts composed, which are composed of, first off, of a stent graft or of grafts comprised of two parts are often complicated in their placement, have a large compressed size, and have imperfections in their support mechanisms and at their point of contact with the vascular wall. These result in the appearance of immediate or future complications or in the inability of placement for a sufficient number of circumstances.

Furthermore, it is a device that, when combined with the fact that it has a limited number of parts, can serve a large number of circumstances of different anatomical dimensions.

A device having a limited number of parts is known from WILSON G J et al: "A Self-expanding bifurcated endovascular graft for abdominal aortic aneurysm repair" vol. 42, no 5, September 1996, pages M386-M393, XP 000 683 606.

According to the present invention, there is provided a graft arrangement for repairing an aortic aneurysm, the arrangement comprising a main graft, to be percutaneously introduced into the aorta via an iliac artery, the main graft being expandable and having a proximal orifice to be located in a part of the aorta adjacent to the renal arteries, the main graft also having a distal orifice end which when expanded serves to receive the proximal ends of a pair of expandable iliac artery grafts, wherein each graft comprises an expandable stent and at least one cover over and/or in the start, and wherein the cross-sectional area of the said distal orifice when expanded is sufficiently less than the sum of the cross-sectional areas of the proximal ends of the iliac artery grafts when expanded so as to form a seal with the said distal orifice when the pair of grafts are expanded therein.

The main graft can be selected to be of a length to extend from the said part to the bifurcation region wherein the aorta extends into the iliac arteries. Alternatively, the main graft can be of a length such that it extends only across the said part. The part of the main graft in the region of the distal orifice is reinforced in order to support the iliac artery grafts when expanded. It is preferred for these stents to be self expanding. The cover at the distal end of the main graft preferably extends beyond the distal orifice so that after the iliac grafts have been inserted and expanded, the extension to the cover enters the interior of the iliac grafts and forms an extra seal therewith. The start of the main graft can comprise hooks or barbs designed to enter the wall of the said part in order to assist in the attachment of that graft to the said part.

### Brief Description of the Drawings

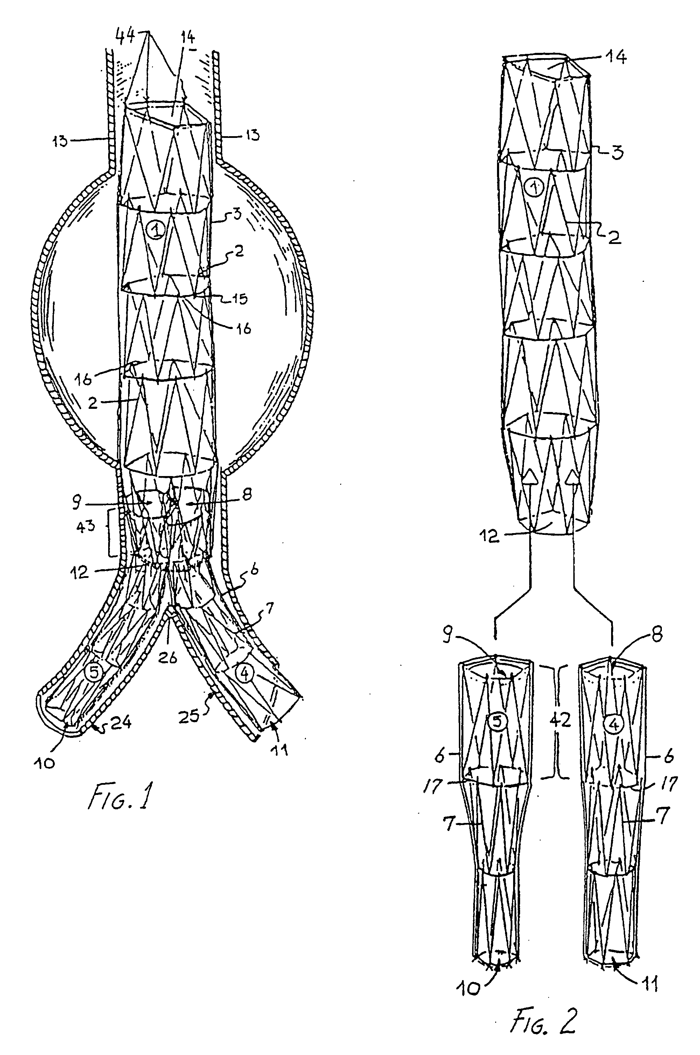

FIG. 1 is a schematic representation of the stent graft after its placement in the aneurysm of the abdominal aorta according to the present technique.
FIG. 2 is a schematic representation of the three parts of the central graft (main cylinder) and the two limbs (peripheral cylinders) from which are composed the stent graft according to the presented technique. The two limbs (peripheral cylinders) enter with their central region (of greater diameter) at the peripheral or distal region of the central cylinder and by their expansion create a leakproof branching of the central graft.

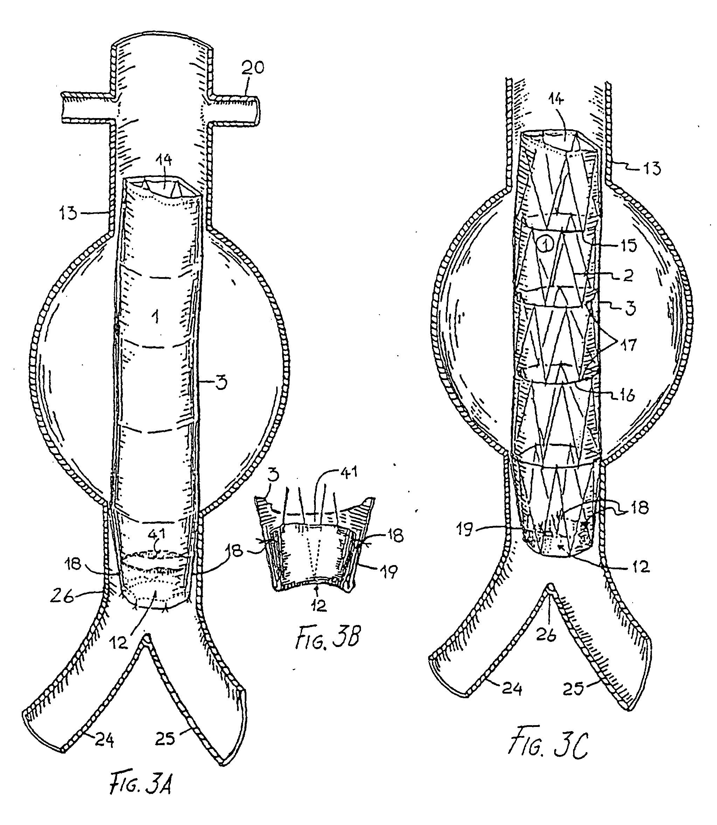

FIG. 3 is a representation of the analytical magnification of the central graft (main cylinder) which is placed in the abdominal aorta in the region between the out-branching of the kidney or renal arteries and the aortic bifurcation after its positioning and its expansion. At the peripheral end can be discerned the refolding and attachment of the thin-walled external covering inside the main cylinder, to reinforce it and to make the branching of the main cylinder more leakage proof after the placement of the two peripheral cylinders.

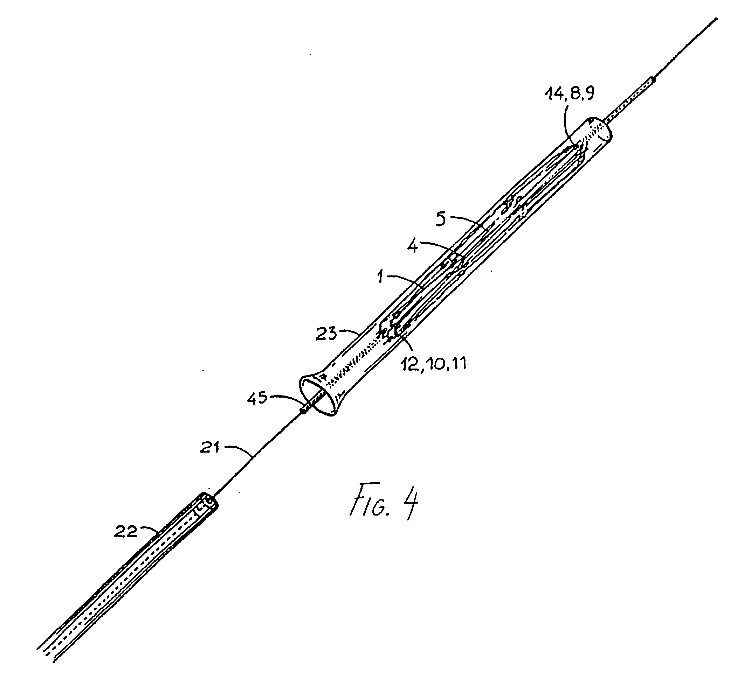

FIG. 4 is a magnified representation of the cylinders in their compressed form inside the storage tubules which are small in diameter equal to the diameter of the placement tubules inside the arteries as well as the propulsion device for the introduction and progression of the graft by means of a guide wire.

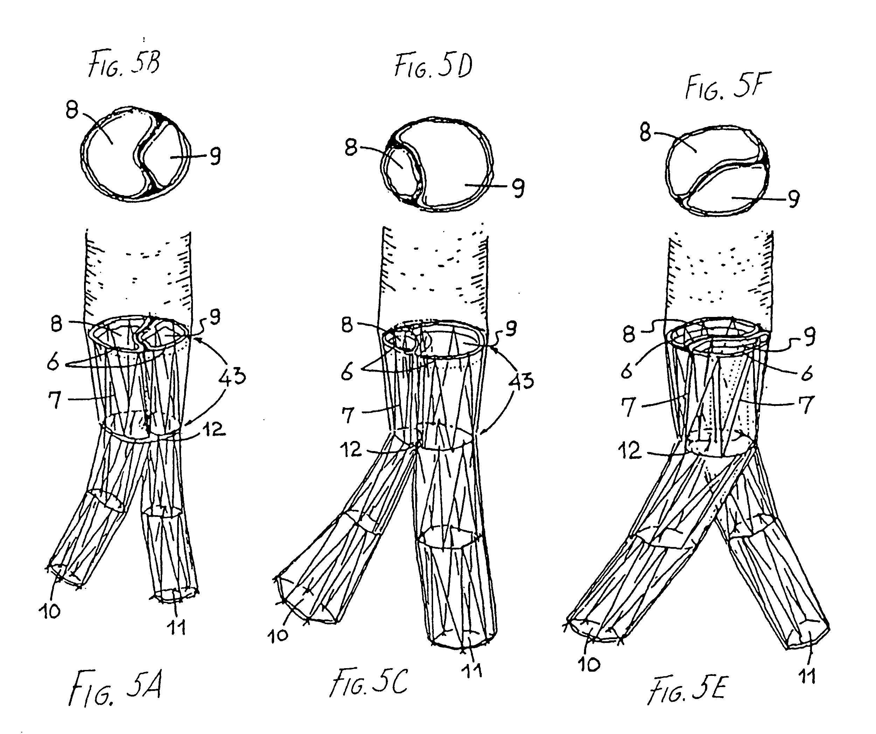

FIG. 5 is a three-dimensional schematic representation of the overlapping regions of the cylinder and of the two peripheral cylinders (limbs) after the expansion of the limbs inside the main cylinder and their leakage-proof application, not only amongst the limbs, but also with the central cylinder due to their self-expanding character. At cross-section, one can clearly see the variety of shapes that the central orifices of the limbs can take when restricted at their external surface by the internal surface of the central cylinders at their overlapping arts.

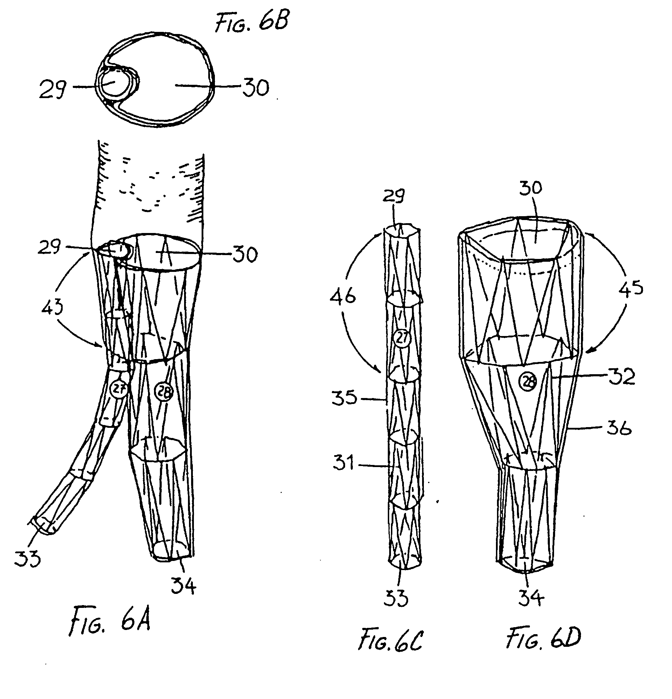

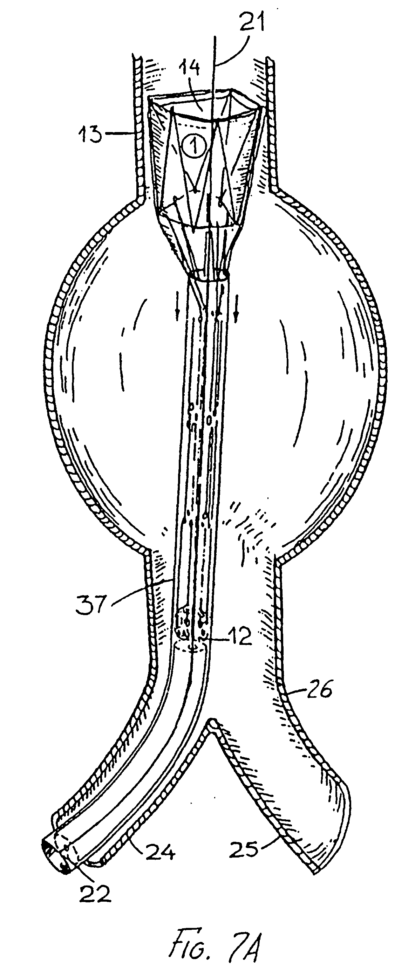

FIG. 6 is a schematic representation of an alternative manner of construction of the limbs of the stent graft where the diameter of the central orifice at one of the limbs at the length of > 2 cm (covered by the central cylindrical part) is equal to the diameter of the peripheral orifice of the main cylinder while the diameter of the other limb is smaller at the center of the orifice and part as well as at the cross-section of the central orifices after the expansion of the two limbs inside the central tube. If they were allowed to expand naturally, the cross-sectional areas of the combined limbs would be twice that of the distal, orifice or the central tube.

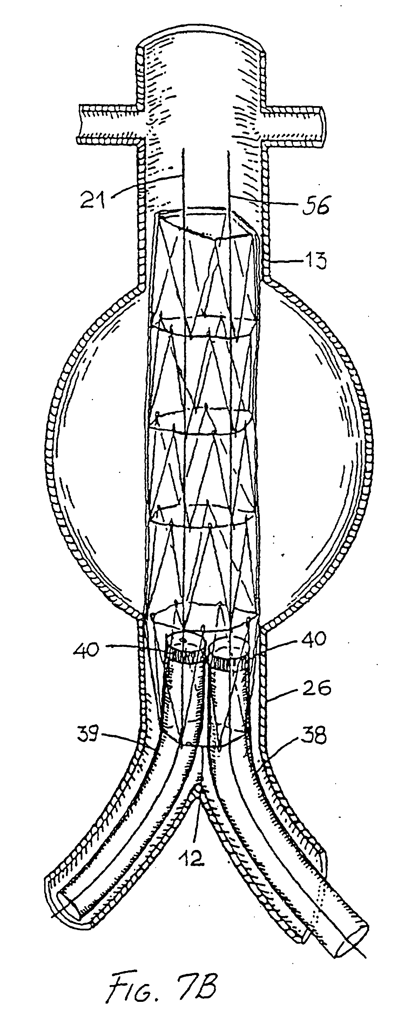

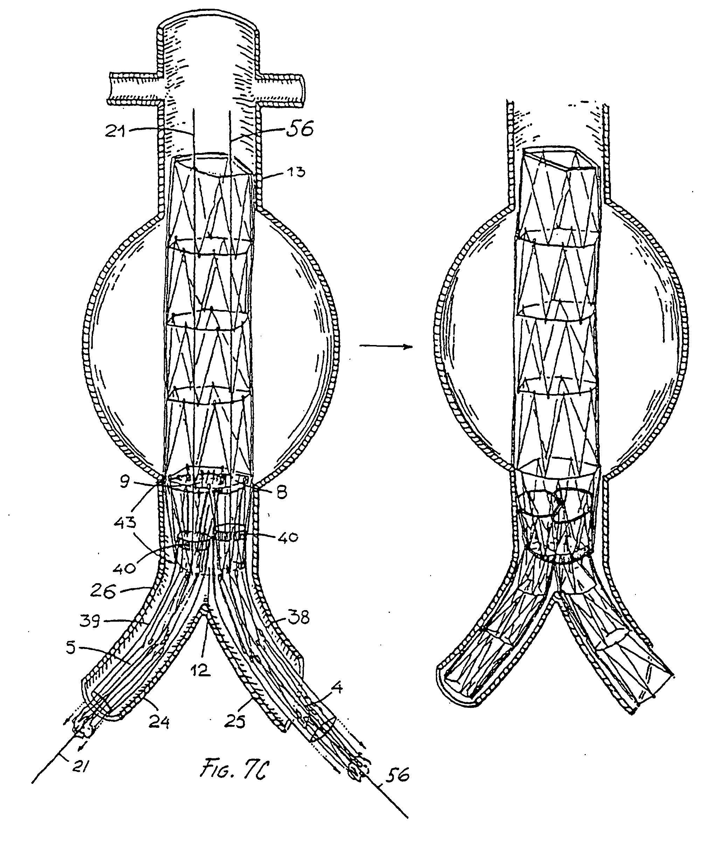

FIG. 7a is a schematic representation of the method of placement of the central graft (main cylinder). This is found compressed inside the placement tubule where it is advanced since the guide wire passes through the graft and is brought to the point of placement with the help of the propulsion device and by the guide wire. The central orifice of the placement tubule has already been advanced more central to the aneurysm and when the graft reaches the position of placement, it is allowed to expand by means of maintaining the propulsion device stable and by means of the peripheral attraction of the placement tubule toward the propulsion device in such a manner so that during its expansion the graft will maintain its position unchanged.
FIG. 7b is a schematic representation after the placement of the central graft a second guide wire is advanced from the other iliac artery and by the peripheral orifice of the main cylinder. On the guide wires of both sides the placement tubules are advanced by an X-ray shadowing ring at their central orifice, through the iliac arteries and through the peripheral orifice of the main cylinder inside the peripheral part of the main cylinder.
FIG. 7c is a schematic representation of the advancement of the two limbs inside the guide introduction tubules using the same technique as that for the placement of the central graft, and of their equal in height positioning by means of retraction of the introduction tubules when these are forwarded at an upright position which is at the same transverse level as the two limbs. The complete release of the limbs (peripheral cylinders) along with the simultaneous or the nonsimultaneous withdrawal of the introduction tubules of both sides results in the fact that the expanded parts of the limbs will come in complete contact with the peripheral nonexpanded healthy region of the corresponding iliac blood vessel at which the flow of blood is directed.

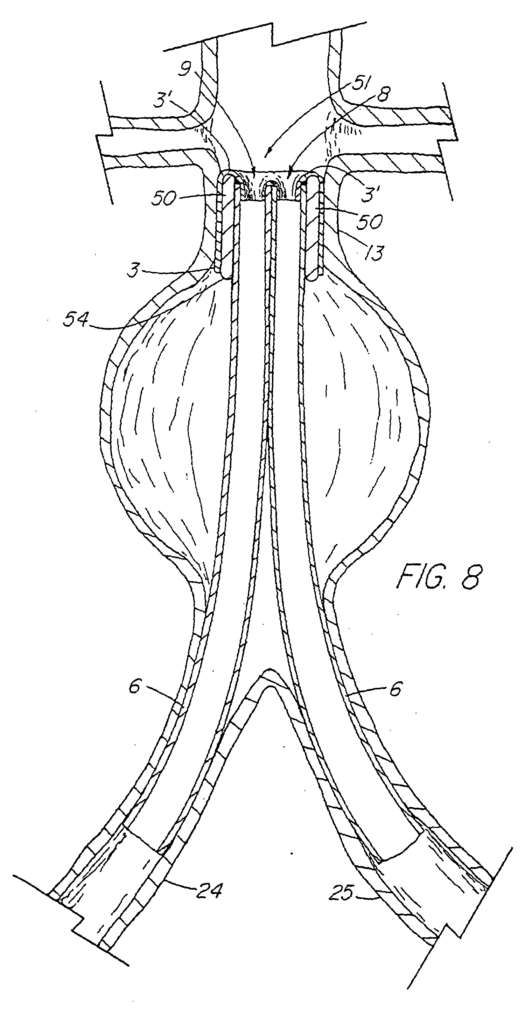

FIG. 8 illustrates an alternative arrangement in which the main graft is substantially contained within the region of the aorta containing the renal arteries. The distal end of the main graft is constructed in a manner similar to that in the other embodiments. The two limbs extend from the iliac arteries into the distal end of the main graft and are sealed in a similar manner.

### Detailed Description

In general, this device presents a stent graft for the therapy of abdominal aortic aneurysms without the need for the presence of healthy peripheral aortic walls (26), whereof the placement of one and only one graft tube would be possible. The need to avoid the pathological wall by airtightness in the nonexpanded part of the region of the iliacs is accommodated by the branching of the central graft (main cylinder) at two peripheral tubes so that the blood can be driven toward both iliac arteries (24, 25) from the central aortic graft. Furthermore, the described graft must have the ability to be compressed to a small starting-off diameter (FIG. 4), such that the advancement and placement in its compressed form is made possible by means of the small diameter tubes (37, 38) inside the vascular lumen from distant regions (from the femoral artery in the abdominal aorta) where released it can regain its original large diameter. This is accomplished in such a way so that it is able to achieve its leakproof perimetric contact with the internal surface of the healthy vascular lumen central and peripheral to an aneurysm. Till today, there have been proposed and used various devices for the accomplishment of the above goals. However these are often complicated and difficult in their usage with the subsequent appearance of complications during and after the operation. To use these devices it is necessary for those performing the operation to acquire lengthy experience and to have ample abilities. Aim of the device which will be described as well as its technical positioning is the simplification of the placement procedure, the minimization of the immediate and future complications and the enhancement of the percentage of successful clinical results with the goal of the possibility of a wide usage of the method of endovascular therapy of aneurysms for those patients where the placement of a stent-graft is necessary.

The herein presented stent-graft is a graft which is comprised of a central "main" cylinder or tube (1) that at the center of the orifice comes into contact with the external surface of the perimeter of the aorta at the level more central to the distended part of the aorta (13) while the periphery of the orifice sits upon the aortic bifurcation (26). The diameter or cross-sectional area of the central or proximal orifice (14) of the main cylinder (1) is equal to or larger than the diameter of the healthy part of the aorta (13) on which the cylinder will be placed. The diameter or cross-sectional area of the peripheral or distal orifice (12) is constant and independent of whatever aortic diameter at the level of its bifurcation (28) where the cylinder will be placed. The length of the main cylinder (1) is determined by whichever length amongst the central point of contact in the aorta (13) and its bifurcation (26).

The main cylinder (1) comprises a stent on skeleton (cast) which is preferably cylindrical and/or metallic with a length that is substantially equal to the distance between the kidney or renal arteries and the aortic bifurcation. This skeleton has as starting-off predetermined diameter α and a compressed diameter β and consists of successive and connected amongst themselves cylindrical pieces of variable length and with a Z configuration made out of biocompatible metal with memory such as stainless steel wire or nitinol (a nickel titanium alloy with thermal memory). The connection of these parts of the skeleton can be accomplished either by sutures (16) which pass through the orifices of the last of the endcrests of each piece or by metallic joints (solderings) in such a manner so as to allow a certain amount of flexibility amongst the various Z parts of the skeleton whilst the length of the skeleton has as minimal as possible changes between its compressed and its starting-off diameter. The present plan of the skeleton is described extensively and consists of the skeleton (2) of the main cylinder (1) which is described in the figures as well as other plans of however, the self-expanding skeletons with similar properties and characteristics which will possibly save the basic idea of the creation of the bifurcation of the herein presented stent-graft. The stent can at its proximal end, have a plurality of barbs or hooks which can, upon expansion, rotate and penetrate part 13 for sealing and securement purposes.

The metallic skeleton or stent of the main cylinder on its outer surface is covered by a tube (3) to form a graft which has a central (14) orifice, and a peripheral or distal (12) orifice. The wall is preferably thin-walled and of PTFE, Dacron, polyurethane or another type of biocompatible plastic. Alternatively, the inner and outer surfaces of the metallic skeleton (2) can be covered by cylindrical tubes (3), or it can be covered on its inner surface only by the cylindrical tube. This tube has a starting-off or proximal diameter which is equal to that of the metallic skeleton and it has a central and peripheral distal orifice and a main body. The central orifice (14) has a diameter which is preferably substantially equal to or greater than that of the healthy part of the aorta at the point of its contact (13) with the main cylinder more central to the aneurysm. The tube(s) of the graft is refolded or is not refolded at the central orifice (14) of the metallic skeleton (2) and is attached upon the central orifice of the skeleton by a series of connective sutures (44) at the end-crests of the central end of the (metallic) skeleton or stent. An outer covering can cover an outer enlargement at the outer proximal end of cylinder (1) to firmly engage part (13) and then a flap can extend inwardly into orifice (14) to improve the seal. The peripheral orifice of the graft has a diameter of 20 - 25 mm and is refolded (18) at a length of 0.5 -1.0 cm at the internal side of the peripheral or distal orifice (12) of the metallic skeleton where it is internally attached by single sutures at two or at three different points of the metallic skeleton (2). Thus the flow of blood after the placement of the main cylinder is accomplished inside the peripheral refolding of the graft of the main cylinder at the peripheral end and creates two or three pockets (petals) (41) minimizing the surface of the peripheral orifice (12) of the main cylinder. Additional attachment points can be used, if desired, to establish additional petals. The main cylinder (1) has a starting-off diameter, which is of the thin-walled covering cylinder (3) around the center (14) and periphery (12) of the orifice end around its body. The metallic skeleton (2) expands the cylinder to this diameter and to a compressed diameter much smaller than that of the original diameter in such a manner so that it can be compressed inside the storage tubule (23) which has a small diameter (FIG. 4) equal to that of the placement tubule (37) which is used for the advancement of the graft inside the blood vessels (FIG. 7a). During the compression of the main cylinder (1) inside the storage tubule (23) it has at its center a catheter (39) which is used for the insertion of the guide wire (21) (which after this it is removed) through the compressed inside the storage tubule main cylinder during the positioning process. The progression of the main cylinder to the placement position is accomplished by its propulsion by the storage tubule (23) to the placement tubule (37), which as previously mentioned, has the same diameter. This is achieved with the help of a propulsion device (22) which moves upon the guide wire (21) which goes through the center of the main cylinder and in continuation through the aneurysm.

The herein presented stent-graft is also comprised of two peripheral cylinders (limbs) (4, 5) which preferably are self-expanding stents or skeletons (7) identical to that of the main cylinder (1) but of different dimensions (a series of Z casts). These are covered at their external surface by a thin-walled cylindrical tube (6), which have an identical or a different composition from that of the main cylinder (1), but are of different dimensions. The peripheral cylinders have central (8, 9) and (peripheral) distal (10, 11) orifices and a starting-off diameter and a compressed diameter such that it becomes possible for them to be placed inside a storage tubule (23) exactly in the same way as with the main cylinder but of a smaller diameter. They can be advanced to the corresponding placement tubules (37, 38) inside of the blood vessels. The thin-walled cylindrical tube (6) covers the entire length of the metallic skeleton (7) and is attached to the central (8, 9) and the peripheral (10, 11) end of the metallic skeleton of each peripheral cylinder. The skeleton (7) of the peripheral cylinders (4, 5) has a diameter in its expanded form equal to or greater than that of the thin-walled cylindrical tube (6) at each of its parts in such a manner so that it comes in complete contact at its external surface with the internal surface of the thin-walled cylindrical tube (6) which has a constant diameter at its expanded form and at each of its parts. The graft can be refolded to a small length < 5mm and can also not be refolded inside the central orifice (8, 9) of the skeleton of each limb and is not refolded at the peripheral orifice (10, 11) of the skeleton of each limb where it is attached by sutures.

The diameter or diameters of the central orifice (8, 9) of each (peripheral cylinders) limb is preferably equal to or approximately up to about 5 mm or more smaller in relation to the diameter of the peripheral orifice (12) of the main cylinder. Alternatively, each orifice (8,9) can be equal to or greater than orifice (12), or each can be significantly smaller than orifice by much more than 5 mm such as 10 or 20 mm. Experimentation of a simple nature can determine sizes of the distal cylinders relative to orifice (12) in order to achieve a sealing effect between cylinders (1, 4 and 5). The diameter of the central orifice of each limb continues at a length of 2 - 2.5 cm at the central part (42) of the graft of each limb which is the length of the first of the Z casts of the preferably self-expanding stents or skeletons (7) of each limb and the point (17) where the first cast of the preferably metallic skeleton is connected to (jointed to) the second cast as has previously been mentioned. This central part (42) of each limb is the part which enters into the distal or peripheral end (43) of the central cylinder for the creation of the bifurcation of the central cylinder as will be mentioned in continuation. The diameter of the peripheral cylinders, more peripheral to the previously mentioned part, has a length and a diameter which vary according to the length and the diameter that is necessary so that the peripheral orifice (10, 11) of each of those two cylinders (4, 5) can come in complete contact with the healthy part of the corresponding iliac blood vessel (24, 25). That is to say, that the peripheral diameter and the length of each limb can differ from each other in relation to the dimensions of the iliac blood vessels of their healthy part and of the length of the damage of each, from the bifurcation of the aorta.

Alternatively, the diameter of the central orifice of the central part of each limb can differ in size (FIG. 6). Specifically, the diameter of the central orifice (30) and of the central part (45) of one of the limbs (28) which enters from the peripheral orifice (12) of the main cylinder (1) is equal to the diameter of the peripheral part and of the orifice (12) of the main cylinder, while the diameter of the central part (46) and the orifice (29) of the other limb (27) can be smaller with the aim of creating a smaller compressed limb diameter and its progression inside of a smaller in diameter placement tubule, percutaneously. In this case, the expanding ability of the metallic skeleton (31) of the smaller in diameter limb is equal to or greater than that of the metallic skeleton (32) of the limb with the greater central diameter. The length as well as the periphery of the peripheral orifice (33, 34) of each peripheral cylinder (limb) (27, 28) can vary as previously mentioned according to the dimensions of the iliac blood vessels and their condition.

As material for the thin-walled covering cylinder (6, 35, 36) of the metallic skeleton (7, 31, 32) of the peripheral cylinders (4, 5, 27, 28) one can use a cylinder made out of thin-walled polytetrafluoroethylene, Dacron™, or another type of biocompatible plastic. This thin-walled cylinder preferably has the previously mentioned constant dimensions of the peripheral cylinders (4, 5, 27, 28) at its noncompressed form as well as after its expansion by the self-expanding metallic skeleton (2) internally. The material of the thin-walled covering cylinder (6, 35, 36) of the metallic skeleton of the peripheral cylinders can cover the cylindrical metallic skeleton at its external surface or at its external and internal surface or at its internal surface.

### Technical Placement - Creation of a Bifurcation

The herein presented stent-graft is created by the placement of three cylinders (1,4,5) (main and two peripheral) of which it is composed and which is executed in the following manner:

After the percutaneous placement of the guide wire (21) from the femoral artery and in a head on direction towards the aorta, an angiogram is performed so as to determine the height of the kidney arteries (20). At this point, as well as at the point of the aortic bifurcation, the X-ray shadowed guided position is monitored by X-rays. On the guide wire is advanced with the help of a diagnostic device, percutaneously, the introduction tubule (37) (sheath) inside of which the main cylinder will be advanced during its placement. The introduction tubule has at its central end an X-ray shadowed ring (40) and at its peripheral end it has a hemostatic valve. During its endovascular advancement, the introduction tubule (37) has inside of it a diagnostic device which makes easier its percutaneous entrance into the artery. The introduction tubule is advanced inside the aorta so far in as necessary so that the X-ray shadowed ring at its central end will be found at a more central level than that of the out-branching of the kidney arteries (20). In continuation, the diagnostic device is removed from inside of the introduction tubule (37). And on the guide wire (21) through the hemostatic valve, the storage tubule (23) is advanced which has a diameter equal to that of the introduction tubule which carries inside its lumen the compressed main cylinder (1) (of the stent graft) that has a length equal to that of the distance amongst the point of the out-branching of the kidney arteries (20) and the aortic bifurcation (26) and a diameter of the central orifice (14) (after its expansion) which is equal to or greater than that of the aorta at the height of the central neck (13) directly underneath the kidney tubules.

The main cylinder (1) is advanced from the storage tubule (23) to the introduction tubule (37) and through this to the placement point with the help of a propulsion column (propulsion device) (22), which passes through the center of the compressed main cylinder. When the main cylinder (1) which casts an X-ray shadow in its entire length (metallic skeleton) is advanced inside the introduction tubule (37) between the guide points that have been placed at the out-branching of the kidney arteries (20) and the aortic bifurcation (26), the propulsion column (22) is maintained in a stable condition by its central end which restrains the peripheral end (12) of the main cylinder at the height of the aortic bifurcation. The introduction tubule (37) is retracted over the column (22) in a centrifugal direction in such a manner so that the main cylinder (1) is progressively released from the introduction tubule at its entire length and will expand (FIG. 7a). When it comes into contact with the internal surface (13) of the aorta directly beneath the kidney arteries (20) it will become more rounder whilst the peripheral orifice sits upon the aortic bifurcation (26). In this way, the dislocation of the main cylinder becomes impossible due to the constant length of the metallic skeleton (2) of the main cylinder which is supported by the aortic bifurcation.

In continuation and after the de novo progression of the column inside the introduction tubule (37), it is advanced on the guide wire (21) through the peripheral orifice (12) inside of the main cylinder (1) so that the X-ray shadowing ring (40) will be found 2 - 2.5 cm more central to the peripheral orifice (12) of the main cylinder.

In the same way, one of the two peripheral cylinders (5) is advanced inside of the introduction tubule through the hemostatic valve. In this manner, its central end (orifice) (9) will reach the height of the X-ray shadowing ring (40) of the introduction tubule.

In continuation, percutaneous advancement of the guide wire (56) is achieved from the femoral artery of the other side after its percutaneous injection. The guide wire is centripetally directed with the help of a guide catheter through the iliac artery (25) and through the peripheral orifice of the main cylinder which sits upon the main cylinder which sits upon the aortic bifurcation inside the lumen of the main cylinder. Advanced on the guide wire follows the second introduction tubule (38) with a diagnostic device inside of it and an X-ray shadowing ring (40) at its central orifice. The second introduction tubule (38) is centripetally advanced through the peripheral orifice (12) of the main cylinder and up to the point where the X-ray shadowing ring at its central orifice is found at the same height (2 - 2.5 cm from the peripheral orifice inside the main cylinder) with the X-ray shadowing ring of the main orifice of the introduction tubule (37) of the femur of the other side (FIG. 7b) inside of which is already found the peripheral cylinder of the limb (5) of the other side at its compressed form. Inside the second introduction tubule end with the technique which was previously mentioned, the second peripheral cylinder (limb) (4) is advanced till the point where its central compressed end is at an equal height as that of the X-ray shadowing ring (40) of the introduction tubule (38) inside of which it is advanced as well as with the central end of the compressed peripheral cylinder (5) of the other side.

After they are X-ray monitored, the two compressed peripheral cylinders (limbs) inside of the introduction tubules have a position of equal height, both with their central end, 2-2.5 cm more central and inside of the peripheral orifice of the main cylinder; that is to say, at the point of the union (41) (joint) of the first with the second Z element of their metallic skeleton which has a corresponding length, the introduction tubules are withdrawn simultaneously or nonsimultaneous in a centrifugal or distal direction and the peripheral cylinders are expanded according to the technique which was mentioned previously for the main cylinder.

After the expansion, the two peripheral cylinders, these having a self-expanding skeleton, preferably each with an equal strength of expansion at the end covered by their main cylinder part extended, are compressed due to the greater total diameter of both in relation to the diameter of the peripheral part of the central cylinder by the main cylinder. In this way, they come into leak-proof contact with the internal surface of the wall of the central cylinder, but also between them but however, maintaining the diameter of both central orifices (8, 9) equal to the diameter of the peripheral part (43) of the main cylinder (1). The shape of the central orifice of the two peripheral cylinders can vary, without these, however, coinciding completely due to the equivalent expansive ability of their metallic skeletons (FIG. 5).

Furthermore, the reversal of the external cover (19) of the main cylinder at the peripheral orifice (12) creates an additional valve mechanism at this level which hinders the escape of blood from the microchasms which may occur during the contact amongst the two peripheral cylinders, but also with the internal surface of the peripheral part (43) of the main cylinder.

In this manner, a blood leak-proof (without the escape of blood) bifurcation of the main cylinder (1) is created at two peripheral cylinders (5, 4) with an entrance orifice (9, 8) of variable shape and area.

The peripheral part of each peripheral cylinder (limb) has a length and a diameter of the peripheral orifice which is analogous to those of the corresponding iliac artery (24, 25), so that after its expansion, it will come in complete contact with the internal surface of the healthy part of the wall of the iliac artery.

After the placement of the "stent graft", according to the manner which was previously mentioned, the direction of the flow of blood is achieved inside of the "stent" graft from the height of the kidney arteries through the orifice of the two limbs and more peripheral to these inside of the iliac arteries with the simultaneous exclusion of the systemic arterial pressure and the blood circulation of the pathologically distended wall of the aneurysm of the aorta which also includes the aortic bifurcation.

In the FIG. 8 embodiment, the graft arrangement comprises a main graft 50 which is formed in a manner similar to graft 1 of the other embodiments. It comprises an inner stent and an outer covering 3, the latter extending upward to form a flap 3' which when the assembly is operational, folds into the graft limbs to assist the sealing process.

The main graft 50 is introduced into the part 13 of the aorta with the proximal end 51 of the graft adjacent to the iliac arteries and with the distal end 40 of the graft adjacent to the distal end of the good tissue 13. The graft is expandable to form a force fit within part 13. If desired the graft 50 can be provided with hooks, which upon expansion of graft 50, rotate and embed themselves into part 13 in a known manner. The graft 50 has an internal cross-sectional area at least at end 54, much less than the sum of the external cross-sectional areas of the limb members 6. The latter are introduced via their respective iliac arteries in a tube such as 37 and allowed to expand and for the seals with the main cylinder 50 and using the flap 3' of the cover extending beyond the provisional end of the cylinder 50. The latter is quite stable since it is supported by the firm or undiseased part 13 of the aorta and it's position is quite fixed. The round flap can have 2 slits at appropriate positions to facilitate entry into the two respective tubes.

In the above described embodiments, self expanding stents have been employed. If that is not desirable for any reason, then non self expanding stents can be employed, but that would necessitate the use of expansion means such as balloons could be used, such as those employed in angioplasty procedures.

## Claims

1. An endovascular stent graft arrangement (1, 4, 5) for repairing aortic aneurysms, comprising an expandable main stent graft (1) having a first end and a second end, the graft arrangement comprising also a pair of expandable peripheral stent grafts (4, 5) each having a first end and a second end the first end having a central orifice (8, 9), each peripheral stent graft (4, 5) being connectable with the second end of the main stent graft (1) which second end has a central orifice (12), wherein the main stent graft (1) and each peripheral stent graft (4, 5) comprise at least one expandable stent and at least one cover (3) over and/or in the expandable stent, and whereby the first ends of the peripheral stent grafts (4, 5) are to be located within the second end of the main stent graft (1) and to be expanded therein, **characterised in that** the sum of the cross-sectional areas of the orifices (8, 9) of the first ends of the pair of peripheral stent grafts (4, 5) when expanded within the second end of the main graft (1) significantly exceeds the cross-sectional area of the orifice (12) of the second end of the main stent graft (1) when expanded, and **in that** a part of the main stent graft (1) in the region of the second end is reinforced in order to support the peripheral stent grafts (4, 5) when expanded within said part.

2. The graft arrangement of any one of the preceding claims, wherein the material of the cover (3) is one of polytetrafluoroethylene, Dacron™ or another type of biocompatible plastic.

3. The graft arrangement of any one of the preceding claims, wherein the cover (3) at the second end (12) of the main stent graft (1) extends beyond the second end of the main stent graft (1) so that after the peripheral stent grafts (4, 5) have been inserted and expanded within the second end of the main stent graft (1), the extension to the cover (3) enters the interior of the peripheral stent grafts (4, 5) and forms an extra seal therewith.

4. The graft arrangement of claim 3, wherein the length of the part of the cover (3) that extends beyond the second end of the main stent graft (1), and is folded inside the second end of the main stent graft (1), is less than half of the diameter of the orifice (12) of the second end of the main stent graft (1).

5. The graft arrangement of claim 4, wherein the cover (3) is attached to the inner side of the at least one stent of the main graft (1) at two or three places to create pockets to be filled by blood flow after implantation of the graft arrangement in order to provide a leak proof contact between the peripheral stent grafts (4, 5) expanded within the second end of the main stent graft (1), and the second end of the main stent graft (1).

6. The graft arrangement of any one of the preceding claim, wherein at least one expandable stent is self-expandable.

7. The graft arrangement of any one of the preceding claims, wherein the at least one expandable stent of the main stent graft (1) comprises hooks or barbs designed to enter the wall of a vessel of a patient in order to assist in the attachment of the main stent graft (1) to the said wall.

8. The graft arrangement of any one of the preceding claims, wherein the material of the at least one stent is metallic.

9. The graft arrangement of claim 7, wherein the material of the at least one stent is a shape memory alloy.

10. The graft arrangement of claim 8, wherein the material of the at least one stent is stainless steel or a nickel titanium alloy.

11. The graft arrangement of any one of the preceding claims, wherein the stent grafts differ from each other in their material, shape, diameter and/or length.

12. The graft arrangement of any one of the preceding claims, wherein each of the grafts is preferably cylindrical and consists of at least one wire circumscribing the graft and having a Z configuration.

13. The graft arrangement of claim 12, wherein the wires having a Z configuration are attached to each other by a biocompatible circumferential wire or by sutures or soldering the Z wires together at their crests.

14. The graft arrangement of any one of the preceding claims, wherein the diameter of the orifice of the first end (8, 9) of each peripheral stent graft (4, 5) is different from the diameter of the second end (10, 11) of the respective peripheral stent grafts (4, 5).

15. The graft arrangement of any one of the preceding claims, wherein the diameter of the orifice of the first end (14) of the main stent graft (1) is equal to the diameter of the second end (12) of the main stent graft (1).

16. The graft arrangement of claim 1, wherein the orifice of first end (8, 9) of the two peripheral stent grafts (4, 5) have a diameter that is substantially equal when expanded.

17. The graft arrangement of claim 1, wherein the orifice of the first end of each peripheral stent graft (4, 5) has a diameter that is approximately 5 mm less than the diameter of the orifice of its second end.

18. The graft arrangement of any one of the preceding claims, wherein the diameter the orifice of the second end of one of the peripheral stent graft (4, 5) is substantially equal to the diameter of the orifice of the second end (10, 11) of the other peripheral stent graft (4, 5).

19. The graft arrangement of any one of the preceding claims, wherein the diameter of the orifice of the second end of one of the peripheral stent graft (4, 5) is different from the diameter of the orifice of the second end of the other peripheral stent graft (4, 5).

20. The graft arrangement of any one of the preceding claims, wherein the peripheral stent grafts (4, 5) have an equal strength of expansion.

## Patentansprüche

1. Endovaskuläre Stentimplantatanordnung (1, 4, 5) zum Ausbessern eines Aortenaneurysmas mit einem expandierbaren Haupt-Stentimplantat (1) mit einem ersten Ende und einem zweiten Ende, wobei die Implantatanordnung ferner ein Paar expandierbare periphere Stentimplantate (4, 5) jeweils mit einem ersten Ende und einem zweiten Ende umfasst, wobei das erste Ende eine zentrale Öffnung (8, 9) aufweist, wobei jedes periphere Stentimplantat (4, 5) mit dem zweiten, eine zentrale Öffnung (12) aufweisenden Ende des Haupt-Stentimplantats (1) verbunden werden kann, wobei das Haupt-Stentimplantat (1) und jedes periphere Stentimplantat (4, 5) mindestens einen expandierbaren Stent und mindestens eine Abdeckung (3) über und/oder in dem expandierbaren Stent umfassen, und wobei die ersten Enden der peripheren Stentimplantate (4, 5) im zweiten Ende des Haupt-Stentimplantats (1) angeordnet werden und darin expandiert werden sollen, **dadurch gekennzeichnet, dass** die Summe der Querschnittsflächen der Öffnungen (8, 9) der ersten Enden des Paars peripherer Stentimplantate (4, 5) bei der Expansion im zweiten Ende des Hauptimplantats (1) im expandierten Zustand signifikant den Querschnittsbereich der Öffnung (12) des zweiten Endes des Haupt-Stentimplantats (1) überschreitet und dass ein Teil des Haupt-Stentimplantats (1) im Bereich des zweiten Endes verstärkt ist, um die peripheren Stent-Implantate (4, 5) bei der Expansion in diesem Teil zu unterstützen.

2. Implantatanordnung nach Anspruch 1, worin das für die Abdeckung (3) ausgewählte Material Polytetrafluorethylen, Dacron™ oder eine andere Art von biokompatiblem Kunststoff ist.

3. Implantatanordnung nach einem der vorhergehenden Ansprüche, worin die Abdeckung (3) sich am zweiten Ende (12) des Haupt-Stentimplantats (1) über das zweite Ende des Haupt-Stentimplantats (1) hinaus erstreckt, so dass die Ausdehnung zur Abdeckung (3) nach Einführen und Expansion der peripheren Stentimplantate (4, 5) im zweiten Ende des Haupt-Stentimplantats (1) in den Innenraum der peripheren Stentimplantate (4, 5) reicht und damit eine Extradichtung bildet.

4. Implantatanordnung nach Anspruch 3, worin die Länge des Teils der Abdeckung (3), die sich über das zweite Ende des Haupt-Stentimplantats (1) hinaus erstreckt und im zweiten Ende des Haupt-Stentimplantats (1) gefaltet ist, weniger als die Hälfte des Durchmessers der Öffnung (12) des zweiten Endes des Haupt-Stentimplantats (1) beträgt.

5. Implantatanordnung nach Anspruch 4, worin die Abdeckung (3) an der Innenseite des mindestens einen Stents des Hauptimplantats (1) an zwei oder drei Stellen befestigt ist, um Taschen zu bilden, die nach der Implantation der Implantatanordnung vom Blutfluss gefüllt werden, um einen leckdichten Kontakt zwischen den peripheren Stentimplantaten (4, 5), die im zweiten Ende des Haupt-Stentimplantats (1) expandiert sind, und dem zweiten Ende des Haupt-Stentimplantats (1) herzustellen.

6. Implantatanordnung nach einem der vorhergehenden Ansprüche, worin mindestens ein expandierbarer Stent selbst-expandierbar ist.

7. Implantatanordnung nach einem der vorhergehenden Ansprüche, worin der mindestens eine expandierbare Stent des Haupt-Stentimplantats (1) Haken oder Widerhaken umfasst, die in die Wand eines Gefäßes eines Patienten eindringen sollen, um die Befestigung des Haupt-Stentimplantats (1) an dieser Wand zu erleichtern.

8. Implantatanordnung nach einem der vorhergehenden Ansprüche, worin das Material des mindestens einen Stents Metall ist.

9. Implantatanordnung nach Anspruch 7, worin das Material des mindestens einen Stents eine Formgedächtnislegierung ist.

10. Implantatanordnung nach Anspruch 8, worin das Material des mindestens einen Stents Edelstahl oder eine Nickel-Titan-Legierung ist.

11. Implantatanordnung nach einem der vorhergehenden Ansprüche, worin die Stentimplantate sich voneinander in Bezug auf Material, Form, Durchmesser und/oder Länge unterscheiden.

12. Implantatanordnung nach einem der vorhergehenden Ansprüche, worin jedes Implantat vorzugsweise zylinderförmig ist und aus mindestens einem Draht besteht, der das Implantat umschreibt und eine Z-Form aufweist.

13. Implantatanordnung nach Anspruch 12, worin die Z-förmigen Drähte mit einem biokompatiblen Umfangsdraht oder durch Nähte oder durch Zusammenlöten der Z-Drähte an den Spitzen aneinander befestigt sind.

14. Implantatanordnung nach einem der vorhergehenden Ansprüche, worin sich der Durchmesser der Öffnung des ersten Endes (8, 9) jedes peripheren Stentimplantats (4, 5) vom Durchmesser des zweiten Endes (10, 11) der jeweiligen peripheren Stentimplantate (4, 5) unterscheidet.

15. Implantatanordnung nach einem der vorhergehenden Ansprüche, worin der Durchmesser der Öffnung des ersten Endes (14) des Haupt-Stentimplantats (1) dem Durchmesser des zweiten Endes (12) des Haupt-Stentimplantats (1) entspricht.

16. Implantatanordnung nach Anspruch 1, worin die Öffnung des ersten Endes (8, 9) der beiden peripheren Stentimplantate (4, 5) einen Durchmesser aufweisen, der im expandierten Zustand im Wesentlichen gleich ist.

17. Implantatanordnung nach Anspruch 1, worin die Öffnung des ersten Endes jedes peripheren Stentimplantats (4, 5) einen Durchmesser aufweist, der ungefähr 5 mm kleiner ist als der Durchmesser der Öffnung seines zweiten Endes.

18. Implantatanordnung nach einem der vorhergehenden Ansprüche, worin der Durchmesser der Öffnung des zweiten Endes eines der peripheren Stentimplantate (4, 5) dem Durchmesser der Öffnung des zweiten Endes (10, 11) des anderen peripheren Stentimplantats (4, 5) im Wesentlichen entspricht.

19. Implantatanordnung nach einem der vorhergehenden Ansprüche, worin sich der Durchmesser der Öffnung des zweiten Endes eines der peripheren Stentimplantate (4, 5) vom Durchmesser der Öffnung des zweiten Endes des anderen peripheren Stentimplantats (4, 5) unterscheidet.

20. Implantatanordnung nach einem der vorhergehenden Ansprüche, worin die peripheren Stentimplantate (4, 5) die gleiche Expansionskraft aufweisen.

## Revendications

1. Disposition de greffe d'endoprothèses vasculaires (1,4,5) pour réparer les anévrismes aortiques, comprenant une endoprothèse greffée principale dilatable (1) ayant une première extrémité et une deuxième extrémité, la disposition d'endoprothèses greffées comprenant également une paire d'endoprothèses greffées périphériques dilatables (4,5) chacun ayant une première extrémité et une deuxième extrémité, la première extrémité ayant un orifice central (8,9), chaque endoprothèse greffée périphérique (4,5) pouvant être raccordée à la deuxième extrémité de l'endoprothèse greffée principale (1), laquelle deuxième extrémité possède un orifice central (12), disposition dans laquelle l'endoprothèse greffée principale (1) et chaque endoprothèse greffée périphérique (4, 5) comprennent au moins une endoprothèse dilatable et au moins une couverture (3) sur et/ou dans l'endoprothèse dilatable, et les premières extrémités des endoprothèses greffées périphériques (4,5) devant ainsi être situées dans la deuxième extrémité de l'endoprothèse greffée principale (1) et s'y dilater, **caractérisée en ce que** la somme des aires de section des orifices (8,9) des premières extrémités de la paire d'endoprothèses greffées périphériques (4,5) lorsqu'elles sont dilatées dans la deuxième extrémité de l'endoprothèse greffée principale (1) dépasse de manière significative l'aire de section de l'orifice (12) de la deuxième extrémité de l'endoprothèse greffée principale (1) lorsqu'elles sont dilatées, et **en ce qu'**une partie de l'endoprothèse greffée principale (1) dans la région de la deuxième extrémité est renforcée afin de soutenir les endoprothèses greffées périphériques (4,5) lorsqu'elles sont dilatées dans ladite partie.

2. Disposition de greffe selon l'une quelconque des revendications précédentes, dans laquelle la matière de la couverture (3) est une matière à base de polytétrafluoroéthylène, Dacron™, ou d'un autre type de matière plastique biocompatible.

3. Disposition de greffe selon l'une quelconque des revendications précédentes, dans laquelle la couverture (3) au niveau de la deuxième extrémité (12) de l'endoprothèse greffée principale (1) s'étend au-delà de la deuxième extrémité de l'endoprothèse greffée principale (1), de sorte que les endoprothèses greffées périphériques (4,5) ont été insérées et se sont dilatées dans la deuxième extrémité de l'endoprothèse greffée principale (1), l'extension en direction de la couverture (3) entre à l'intérieur des endoprothèses greffées périphériques (4,5) et forme un joint étanche supplémentaire avec ces dernières.

4. Disposition de greffe selon la revendication 3, dans laquelle la longueur de la partie de la couverture (3) qui s'étend au-delà de la deuxième extrémité de l'endoprothèse greffée principale (1) et qui est pliée à l'intérieur de la deuxième extrémité de l'endoprothèse greffée principale (1), est inférieure à la moitié du diamètre de l'orifice (12) de la deuxième extrémité de l'endoprothèse greffée principale (1).

5. Disposition de greffe selon la revendication 4, dans laquelle la couverture (3) est fixée au côté intérieur de ladite au moins une endoprothèse greffée principale (1) en deux ou trois endroits, afin de créer des poches destinées à être remplies par un flux sanguin après implantation de la disposition de greffe, afin de procurer un contact étanche entre les endoprothèses greffées périphériques (4,5) dilatées à l'intérieur de la deuxième extrémité de l'endoprothèse greffée principale (1), et la deuxième extrémité de l'endoprothèse greffée principale (1).

6. Disposition de greffe selon l'une quelconque des revendications précédentes, dans laquelle au moins une endoprothèse dilatable est auto-dilatable.

7. Disposition de greffe selon l'une quelconque des revendications précédentes, dans laquelle ladite au moins une endoprothèse dilatable de l'endoprothèse greffée principale (1) comprend des crochets ou des barbillons conçus en vue d'entrer dans la paroi d'un vaisseau d'un patient afin de contribuer à la fixation de l'endoprothèse greffée principale (1) à ladite paroi.

8. Disposition de greffe selon l'une quelconque des revendications précédentes, dans laquelle la matière de ladite au moins une endoprothèse est métallique.

9. Disposition de greffe selon la revendication 7, dans laquelle la matière de ladite au moins une endoprothèse est un alliage à mémoire de forme.

10. Disposition de greffe selon la revendication 8, dans laquelle la matière de ladite au moins une endoprothèse est l'acier inoxydable ou un alliage de nickel-titane.

11. Disposition de greffe selon l'une quelconque des revendications précédentes, dans laquelle les endoprothèses greffées diffèrent l'une de l'autre dans leur matière, forme, diamètre et/ou longueur.

12. Disposition de greffe selon l'une quelconque des revendications précédentes, dans laquelle chacune des endoprothèses est de préférence cylindrique et se compose d'au moins un fil circonscrivant l'endoprothèse et présentant une configuration en Z.

13. Disposition de greffe selon la revendication 12, dans laquelle les fils ayant une configuration en Z sont fixés l'un à l'autre à l'aide d'un fil circonférentiel biocompatible, ou bien à l'aide de sutures ou par soudage mutuel des fils en Z au niveau de leurs crêtes.

14. Disposition de greffe selon l'une quelconque des revendications précédentes, dans laquelle le diamètre de l'orifice de la première extrémité (8,9) de chaque endoprothèse greffée périphériques (4,5) est différent du diamètre de la deuxième extrémité (10,11) des endoprothèses périphériques (4,5).

15. Disposition de greffe selon l'une quelconque des revendications précédentes, dans laquelle le diamètre de l'orifice de la première extrémité (14) de l'endoprothèse greffée principale (1) est égal au diamètre de la deuxième extrémité (12) de l'endoprothèse greffée principale (1).

16. Disposition de greffe selon la revendication 1, dans laquelle l'orifice de la première extrémité (8,9) des deux endoprothèses périphériques (4,5) a un diamètre qui est sensiblement égal lorsqu'elles se dilatent.

17. Disposition de greffe selon la revendication 1, dans laquelle l'orifice de la première extrémité de chaque endoprothèse greffée périphérique (4,5) a un diamètre qui est inférieur d'environ 5 mm au diamètre de l'orifice de sa deuxième extrémité.

18. Disposition de greffe selon l'une quelconque des revendications précédentes, dans laquelle le diamètre de l'orifice de la deuxième extrémité de l'une des endoprothèses greffées périphériques (4,5) est sensiblement égal au diamètre de l'orifice de la deuxième extrémité (10,11) de l'autre endoprothèse greffée périphérique (4,5).

19. Disposition de greffe selon l'une quelconque des revendications précédentes, dans laquelle le diamètre de l'orifice de la deuxième extrémité de l'une des endoprothèses périphériques (4,5) est différent du diamètre de l'orifice de la deuxième extrémité de l'autre endoprothèse greffée périphérique (4,5).

20. Disposition de greffe selon l'une quelconque des revendications précédentes, dans laquelle les endoprothèses greffées périphériques (4,5) ont une force de dilatation égale.
